(19) Europäisches Patentamt
European Patent Office
Office européen des brevets

(11) EP 2 780 044 B1

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(21) Anmeldenummer: **12786983.2**

(22) Anmeldetag: **13.11.2012**

(51) Int Cl.:
***A61L 15/22*** (2006.01) ***A61L 15/60*** (2006.01)
***C08J 3/24*** (2006.01) ***A61L 15/24*** (2006.01)
***B01J 20/26*** (2006.01) *C08L 33/08* (2006.01)

(86) Internationale Anmeldenummer:
**PCT/EP2012/072493**

(87) Internationale Veröffentlichungsnummer:
**WO 2013/072311 (23.05.2013 Gazette 2013/21)**

(54) **VERFAHREN ZUR HERSTELLUNG THERMISCH OBERFLÄCHENNACHVERNETZTER WASSERABSORBIERENDER POLYMERPARTIKEL**

METHOD FOR PRODUCING THERMALLY SURFACE CROSSLINKED WATER-ABSORBENT POLYMER PARTICLES

PROCÉDÉ POUR LA PRODUCTION DES PARTICULES POLYMÈRES POST-RÉTICULÉES THERMIQUEMENT EN SURFACE QUI ABSORBENT L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.11.2011 EP 11189744**

(43) Veröffentlichungstag der Anmeldung:
**24.09.2014 Patentblatt 2014/39**

(73) Patentinhaber: **BASF SE**
**67056 Ludwigshafen am Rhein (DE)**

(72) Erfinder:
- **BELACK, Jörg**
**55432 Damscheid (DE)**
- **DANIEL, Thomas**
**67165 Waldsee (DE)**
- **BAUDUIN, Christophe**
**68723 Plankstadt (DE)**

(74) Vertreter: **BASF IP Association**
**BASF SE**
**ZRX-C6**
**67056 Ludwigshafen (DE)**

(56) Entgegenhaltungen:
**WO-A1-2006/025586 US-B1- 6 239 230**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 780 044 B1

## Beschreibung

Die vorliegende Erfindung betrifft ein Verfahren zur Herstellung thermisch oberflächennachvernetzter wasserabsorbierender Polymerpartikel, wobei die wasserabsorbierenden Polymerpartikel vor, während oder nach der thermischen Oberflächennachvernetzung mit mindestens einem Komplex, bestehend aus einem mehrwertigen Metallsalz und einem 2-Hydroxycarbonsäureamid, beschichtet werden.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymere werden oft auch als "absorbent resin", "superabsorbents ", "superabsorbent polymer", "absorbent polymer", "absorbent gelling material", "hydrophilic polymer", "Hydrogele" oder "Superabsorber" bezeichnet.

Die Herstellung wasserabsorbierender Polymere wird in der Monographie "Modern Superabsorbent Polymer Technology", F.L. Buchholz und A.T. Graham, Wiley-VCH, 1998, Seiten 71 bis 103, beschrieben.

Zur Verbesserung der Anwendungseigenschaften, wie beispielsweise Flüssigkeitsleitfähigkeit in der Windel und Absorptionskapazität unter Druck, werden wasserabsorbierende Polymerpartikel im allgemeinen oberflächennachvernetzt. Diese Oberflächennachvernetzung kann in wässriger Gelphase durchgeführt werden. Vorzugsweise werden aber getrocknete, gemahlene und klassierte Polymerpartikel (Grundpolymer) an der Oberfläche mit einem Oberflächennachvernetzer beschichtet und thermisch oberflächennachvernetzt. Dazu geeignete Vernetzer sind Verbindungen, die mindestens zwei Gruppen enthalten, die mit den Carboxylatgruppen der wasserabsorbierenden Polymerpartikel kovalente Bindungen bilden können.

Die Bestimmung der Flüssigkeitsleitfähigkeit kann beispielsweise über die Gelbettpermeabilität (GBP) gemäß US 2005/0256757 durchgeführt werden.

WO 2004/069915 A2 beschreibt ein Verfahren zur Herstellung wasserabsorbierender Polymerpartikel mit hoher Flüssigkeitsweiterleitung (SFC), die gleichzeitig über eine starke Dochtwirkung verfügen, d.h. die wässrige Flüssigkeiten gegen die Schwerkraft aufsaugen können. Die Dochtwirkung der Polymerpartikel wird durch eine spezielle Oberflächenbeschaffenheit erreicht.

Dazu werden Partikel mit einer Größe von weniger als 180 μm aus dem Grundpolymer ausgesiebt, agglomeriert und mit den vorher abgetrennten Partikeln größer 180 μm vereinigt.

WO 2009/041731 A1 lehrt zur Verbesserung von Flüssigkeitsweiterleitung (SFC) und Zentrifugenretentionskapazität (CRC) die Beschichtung mit mehrwertigen Metallkationen und Fettsäuren. Fettsäuren senken allerdings auch die Oberflächenspannung des wässrigen Extrakts der wasserabsorbierenden Polymerpartikel und erhöhen somit das Risiko des Auslaufens der Windel.

US 2010/0247916 offenbart die Verwendung basischer Salze mehrwertiger Metallkationen, insbesondere zur Verbesserung von Gelbettpermeabilität (GBP) und Absorption unter einem Druck von 49,2 g/$cm^2$ (AUL0.7psi).

Für ultradünne Hygieneartikel werden vorzugsweise wasserabsorbierende Polymerpartikel ohne grobe Körner (Partikel) benötigt, da diese fühlbar wären und beim Verbraucher auf Ablehnung stoßen können. Jedoch kann es aus Wirtschaftlichkeitsgründen erforderlich sein die gesamte Windelkonstruktion in der Optimierung der Korngrößenverteilung der wasserabsorbierenden Polymerpartikel zu berücksichtigen. Eine gröbere Korngrößenverteilung kann zu einem besseren Verhältnis von Absorptionskapazität und Flüssigkeitsleitfähigkeit in der Windel führen, jedoch muss dazu üblicherweise eine geeignete faserige Flüssigkeitsverteilschicht auf dem absorbierenden Kern aufgelegt werden bzw. muss das raue Pulver auch von hinten mit einem weichen Vlies abgedeckt werden.

In ultradünnen Hygieneartikeln spielt dies eine wichtige Rolle, da diese absorbierende Kerne enthalten können, welche zu 50 bis 100 Gew.-% aus wasserabsorbierenden Polymerpartikeln bestehen, so dass die Polymerpartikel im Gebrauch sowohl die Speicherfunktion für die Flüssigkeit übernehmen als auch den aktiven (Dochtwirkung) und passiven Flüssigkeitstransport (Flüssigkeitsleitfähigkeit). Je mehr Zellstoff durch wasserabsorbierende Polymerpartikel oder Synthesefasern ersetzt wird, desto mehr Transportfunktionen müssen die wasserabsorbierenden Polymerpartikel zusätzlich zu ihrer Speicherfunktion erfüllen.

Ein Gegenstand der vorliegenden Erfindung ist daher die Bereitstellung geeigneter wasserabsorbierender Polymerpartikel für Hygieneartikel die zumindest stellenweise im absorbierenden Kern oder im gesamten absorbierenden Kern eine Konzentration an wasserabsorbierenden Polymerpartikeln von mindestens 50 Gew.-%, bevorzugt mindestens 60 Gew.-%, mehr bevorzugt mindestens 70 Gew.-%, noch mehr bevorzugt mindestens 80 Gew.-%, am meisten bevorzugt von 90 bis 100 Gew.-% enthalten. Der absorbierende Kern ist der Teil des Hygieneartikels, der für die Speicherung und Rückhaltung der zu absorbierenden wässrigen Körperflüssigkeit dient. Er besteht üblicherweise oft aus einem Gemisch von Fasern, beispielsweise Zellstoff, und den darin verteilten wasserabsorbierenden Polymerpartikeln. Optional können noch Binder und Kleber eingesetzt werden um den absorbierenden Kern zusammenzuhalten. Alternativ können die

wasserabsorbierenden Polymerpartikel auch in Taschen zwischen mindestens zwei miteinander verbundenen Vliesen eingeschlossen sein. Die übrigen Bestandteile des Hygieneartikels, auch die optionale Umhüllung und Abdeckung des absorbierenden Kerns, wird im Sinne dieser Erfindung nicht zum absorbierenden Kern gehörend gezählt.

Zur Herstellung derartiger wasserabsorbierender Polymerpartikel werden üblicherweise Beschichtungen multivalenter Kationen eingesetzt. Besonders geeignet sind lösliche Salze mehrwertiger Metallkationen wie Aluminiumsulfat, Polyamine und wasserunlösliche Phospate mehrwertiger Metallkationen wie Kalzium, Zirkonium, Eisen und Aluminium.

Die Salze mehrwertiger Metallkationen, insbesondere des Aluminiums, Zirkoniums und des Eisens, sind zwar gut geeignet die gewünschten Effekte auf die Flüssigkeitsleitfähigkeit zu erzielen, aber der Erfolg hängt vom vorhandenen Anion ab. Wenn beispielsweise Aluminiumsulfat eingesetzt wird, kommt es schon bei der Beschichtung der wasserabsorbierenden Polymerpartikel leicht zur Klumpen- oder Staubbildung, außerdem wird die Absorptionskapazität unter Druck verringert. Der Einsatz von Aluminiumlaktat kann ebenfalls zu Staubproblemen führen und außerdem wirkt die bei der Beschichtung der wasserabsorbierenden Polymerpartikel frei vorliegende Milchsäure stark korrosiv. Die Milchsäure kann weiterhin beim Aufkonzentrieren durch Wasserentzug nach der Beschichtung zu Polymilchsäure kondensieren, welche die Oberfläche der damit beschichteten wasserabsorbierenden Polymerpartikel unerwünscht klebrig machen kann. Dadurch können die Fließeigenschaften der wasserabsorbierenden Polymerpartikel beeinträchtigt werden.

Andere Aluminiumsalze oder Salze mehrwertiger Kationen mit vielen organischen Anionen wirken entweder nicht in gewünschter Weise oder sind schwer löslich und weisen damit keine Vorteile gegenüber den oben beschriebenen wasserunlöslichen Phosphaten auf.

Es bestand daher die Aufgabe wasserabsorbierende Polymerpartikel mit hoher Zentrifugenretentionskapazität (CRC) und hoher Gelbettpermeabilität (GBP) bereitzustellen.

Es bestand weiterhin die Aufgabe geeignete Beschichtungen für wasserabsorbierende Polymerpartikel zur Verfügung zu stellen, die leicht aufzubringen sind, keine Staub- oder Klebrigkeitsproblematik aufweisen und nicht übermäßig zu Korrosion im Herstellverfahren der wasserabsorbierenden Polymerpartikel führen.

Es bestand weiterhin die Aufgabe geeignete Beschichtungen für wasserabsorbierende Polymerpartikel zur Verfügung zu stellen, die leicht aus wässriger Lösung aufzubringen sind und keine Anwendungsprobleme wegen wenig oder unlöslicher Salze multivalenter Kationen aufweisen.

Die Aufgabe wird gelöst durch Bereitstellung wasserabsorbierender Polymerpartikel, enthaltend

a) mindestens ein polymerisiertes ethylenisch ungesättigtes, säuregruppentragendes Monomer, das zumindest teilweise neutralisiert sein kann,
b) mindestens einen polymerisierten Vernetzer,
c) optional ein oder mehrere mit den unter a) genannten Monomeren copolymerisierte ethylenisch ungesättigte Monomere,
d) optional ein oder mehrere wasserlösliche Polymere, und
e) mindestens einen umgesetzten Oberflächennachvernetzer,

wobei die wasserabsorbierenden Polymerpartikel mit mindestens einem Komplex, bestehend aus einem mehrwertigen Metallsalz der allgemeinen Formel (I)

$$M^n(X)_a(OH)_b \qquad (I),$$

und einem 2-Hydroxycarbonsäureamid, beschichtet sind, worin

M mindestens ein mehrwertiges Metallkation,
X mindestens ein Säureanion,
a eine Zahl von 0 bis n/m, wobei m die Anzahl der negativen Ladungen des Säureanions und n die Anzahl der positiven Ladungen des mehrwertigen Metallkations ist, und
b eine Zahl 0 bis n

bedeuten.

Geeignete Säureanionen X sind beispielsweise Anionen von Säuren ausgewählt aus der Gruppe Glyzerinsäure, Zitronensäure, Glykolsäure, Milchsäure, Lactoylmilchsäure, Malonsäure, Hydroxymalonsäure, Weinsäure, Glyzerin-1,3-di-phosphorsäure, Glycerinmonophosphorsäure, Essigsäure, Ameisensäure, Propionsäure, Methansulfonsäure und Schwefelsäure. Bevorzugt sind Anionen der Essigsäure, Propionsäure, Glykolsäure, Milchsäure, Methansulfonsäure und Schwefelsäure.

Die mehrwertigen Metallsalze der allgemeinen Formel (I) können aber auch reine Hydroxide mehrwertiger Metallkationen sein.

Geeignete mehrwertige Metallkationen sind die Kationen des Aluminiums, Zirkoniums, Eisens, Titans, Zinks, Kalziums, Magnesiums und Strontiums. Bevorzugt sind die Kationen des Aluminiums, Zirkoniums, Titans und Eisens, mehr bevorzugt sind die Kationen des Aluminiums, Titans und Zirkoniums, am meisten bevorzugt ist das Kation des Aluminiums.

In einer erfindungsgemäß besonders bevorzugten Ausführung wurde reines Aluminiumhydroxid eingesetzt.

Geeignete 2-Hydroxycarbonsäureamide sind die Amide der Äpfelsäure, Glykolsäure, Isozitronensäure, Mandelsäure, Milchsäure, Tartronsäure, Weinsäure und Zitronensäure. Bevorzugt sind Glykolsäureamid und Milchsäureamid, am meisten bevorzugt ist Milchsäureamid.

Der Komplex aus einem mehrwertigen Metallsalz der allgemeinen Formel (I) und einem 2-Hydroxycarbonsäureamid enthält vorzugsweise von 0,1 bis 3 mol, besonders bevorzugt von 0,3 bis 2 mol, ganz besonders bevorzugt von 0,5 bis 1,5 mol, des 2-Hydroxycarbonsäureamids, jeweils bezogen auf das mehrwertige Metallkation.

Die erfindungsgemäßen wasserabsorbierenden Polymerpartikel enthalten vorzugsweise 0,001 bis 5 Gew.-%, besonders bevorzugt 0,01 bis 2 Gew.-%, ganz besonders bevorzugt 0,1 bis 1 Gew.-%, des Komplexes aus einem mehrwertigen Metallsalz und einem 2-Hydroxycarbonsäureamid.

Der Neutralisationsgrad des polymerisierten Monomere a) kann von 0 bis 100 mol-% variieren, üblicherweise liegt er im Bereich 30 bis 90 mol-%. Um die erfindungsgemäße Aufgabe zu erfüllen kann es aber notwendig sein, den Neutralisationsgrad so zu wählen, dass eine optimale Absorptionskapazität mit guter Flüssigkeitsleitfähigkeit kombiniert wird. Daher sind die Säuregruppen des polymerisierten Monomeren a) vorzugsweise zu größer 45 mol-%, bevorzugt zu größer 55 mol-%, besonders bevorzugt zu größer 65 mol-%, ganz besonders bevorzugt zu größer 68 mol-%, und vorzugsweise zu höchstens 80 mol-%, bevorzugt zu höchstens 76 mol-%, besonders bevorzugt zu höchstens 74 mol-%, ganz besonders bevorzugt zu höchstens 72 mol-%, neutralisiert.

Geeignete Monomere für das polymerisierte Monomer a), den polymerisierten Vernetzer b) und das polymerisierte Monomer c) sind die unten beschriebenen Monomere i), Vernetzer ii) und Monomere iii).

Geeignete wasserlösliche Polymere für das wasserlösliche Polymere d) sind die unten beschriebenen wasserlöslichen Polymere iv).

Geeignete Oberflächennachvernetzer für die umgesetzten Oberflächennachvernetzer e) sind die unten beschriebenen Oberflächennachvernetzer v).

Die wasserabsorbierenden Polymerpartikel weisen üblicherweise eine Partikelgröße bis höchstens 1000 µm auf, vorzugsweise liegt die Partikelgröße unter 900 µm, bevorzugt unter 850 µm, mehr bevorzugt unter 800 µm, noch mehr bevorzugt unter 700 µm, am meisten bevorzugt unter 600 µm. Die wasserabsorbierenden Polymerpartikel weisen eine Partikelgröße von mindestens 50 µm, bevorzugt mindestens 100 µm, mehr bevorzugt von mindestens 150 µm, noch mehr bevorzugt von mindestens 200 µm, am meisten bevorzugt von mindestens 300 µm, auf. Die Partikelgröße kann gemäß der von der EDANA empfohlenen Testmethode Nr. WSP 220.2-05 "Particle Size Distribution" bestimmt werden.

Vorzugsweise weisen weniger als 2 Gew.-%, besonders bevorzugt weniger als 1,5 Gew.-%, ganz besonders bevorzugt weniger als 1 Gew.-%, der wasserabsorbierenden Polymerpartikel eine Partikelgröße von weniger als 150 µm auf.

Vorzugsweise weisen weniger als 2 Gew.-%, besonders bevorzugt weniger als 1,5 Gew.-%, ganz besonders bevorzugt weniger als 1 Gew.-%, der wasserabsorbierenden Polymerpartikel eine Partikelgröße von über 850 µm auf.

Vorzugsweise weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 150 bis 850 µm auf.

In einer bevorzugten Ausführungsform weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 150 bis 700 µm auf.

In einer weiteren bevorzugten Ausführungsform weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 200 bis 700 µm auf.

In einer weiteren mehr bevorzugten Ausführungsform weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 150 bis 600 µm auf.

In einer weiteren noch mehr bevorzugten Ausführungsform weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 200 bis 600 µm auf.

In einer weiteren besonders bevorzugten Ausführungsform weisen mindestens 90 Gew.-%, bevorzugt mindestens 95 Gew.-%, besonders bevorzugt mindestens 98 Gew.-%, ganz besonders bevorzugt mindestens 99 Gew.-%, der wasserabsorbierenden Polymerpartikel eine Partikelgröße von 300 bis 600 µm auf.

Der Wassergehalt der erfindungsgemäßen wasserabsorbierenden Polymerpartikel beträgt vorzugsweise weniger als 6 Gew.-%, besonders bevorzugt weniger als 4 Gew.-%, ganz besonders bevorzugt weniger als 3 Gew.-%. Höhere Wassergehalte sind selbstverständlich auch möglich, verringern aber typischerweise die Absorptionskapazität und werden daher nicht bevorzugt.

Die Oberflächenspannung des wässrigen Extrakts des gequollenen wasserabsorbierenden Polymerpartikel bei 23°C beträgt üblicherweise mindestens 0,05 N/m, vorzugsweise mindestens 0,055 N/m, bevorzugt mindestens 0,06 N/m, besonders bevorzugt mindestens 0,065 N/m, ganz besonders bevorzugt mindestens 0,068 N/m.

Die Zentrifugenretentionskapazität (CRC) der wasserabsorbierenden Polymerpartikel beträgt üblicherweise mindestens 24 g/g, vorzugsweise mindestens 26 g/g, bevorzugt mindestens 28 g/g, besonders bevorzugt mindestens 30 g/g, ganz besonders bevorzugt mindestens 34 g/g, und üblicherweise nicht über 50 g/g.

Die Absorption unter einem Druck von 49,2 g/cm$^2$ (AUL0.7psi) der wasserabsorbierenden Polymerpartikel beträgt üblicherweise mindestens 15 g/g, vorzugsweise mindestens 17 g/g, bevorzugt mindestens 20 g/g, besonders bevorzugt mindestens 22 g/g, ganz besonders bevorzugt mindestens 24 g/g, und üblicherweise nicht über 45 g/g.

Die Gelbettpermeabilität (GBP) der wasserabsorbierenden Polymerpartikel beträgt beispielsweise mindestens 10 Darcies, typischerweise mindestens 15 Darcies, vorzugsweise mindestens 20 Darcies, bevorzugt mindestens 25 Darcies, besonders bevorzugt mindestens 30 Darcies, ganz besonders bevorzugt mindestens 35 Darcies, und üblicherweise nicht über 200 Darcies.

Bevorzugte erfindungsgemäße wasserabsorbierende Polymerpartikel sind Polymerpartikel mit den obengenannten Eigenschaften.

Ein weiterer Gegenstand der vorliegenden Erfindung ist ein Verfahren zur Herstellung wasserabsorbierender Polymerpartikel durch Polymerisation einer Monomerlösung oder -suspension, enthaltend

i) mindestens ein ethylenisch ungesättigtes, säuregruppentragendes Monomer, das zumindest teilweise neutralisiert sein kann,
ii) mindestens einen Vernetzer,
iii) optional ein oder mehrere mit den unter i) genannten Monomeren copolymerisierbare ethylenisch ungesättigte Monomere und
iv) optional ein oder mehrere wasserlösliche Polymere,

wobei das dabei erhaltene Polymergel getrocknet, gemahlen, klassiert, mit

v) mindestens einem Oberflächennachvernetzer

beschichtet und thermisch oberflächennachvernetzt wird, dadurch gekennzeichnet, dass die wasserabsorbierenden Polymerpartikel vor, während oder nach der thermischen Oberflächennachvernetzung mit mindestens einem Komplex, bestehend aus einem mehrwertigen Metallsalz der allgemeinen Formel (I)

$$M^n(X)_a(OH)_b \qquad (I),$$

und einem 2-Hydroxycarbonsäureamid, beschichtet werden, worin

M mindestens ein mehrwertiges Metallkation,
X mindestens ein Säureanion,
a eine Zahl von 0 bis n/m, wobei m die Anzahl der negativen Ladungen des Säureanions und n die Anzahl der positiven Ladungen des mehrwertigen Metallkations ist, und
b eine Zahl 0 bis n

bedeuten.

Geeignete Säureanionen X sind beispielsweise Anionen von Säuren ausgewählt aus der Gruppe Glyzerinsäure, Zitronensäure, Glykolsäure, Milchsäure, Lactoylmilchsäure, Malonsäure, Hydroxymalonsäure, Weinsäure, Glyzerin-1,3-di-phosphorsäure, Glycerinmonophosphorsäure, Essigsäure, Ameisensäure, Propionsäure, Methansulfonsäure und Schwefelsäure. Bevorzugt sind Anionen der Essigsäure, Propionsäure, Glykolsäure, Milchsäure, Methansulfonsäure und Schwefelsäure.

Es ist aber auch möglich reine Hydroxide mehrwertiger Metallkationen einzusetzen.

Geeignete mehrwertige Metallkationen sind die Kationen des Aluminiums, Zirkoniums, Eisens, Titans, Zinks, Kalziums, Magnesiums und Strontiums. Bevorzugt sind die Kationen des Aluminiums, Zirkoniums, Titans und Eisens, mehr bevorzugt sind die Kationen des Aluminiums, Titans und Zirkoniums, am meisten bevorzugt ist das Kation des Aluminiums.

In einer erfindungsgemäß besonders bevorzugten Ausführung wird reines Aluminiumhydroxid eingesetzt.

Die mehrwertigen Metallsalze der allgemeinen Formel (I) können durch Umsetzung eines Hydroxids, beispielsweise Aluminiumhydroxid oder Natriumaluminat, mit mindestens einer Säure, beispielsweise Schwefelsäure, hergestellt werden. Die Umsetzung erfolgt bevorzugt in wässriger Lösung oder Dispersion.

Geeignete 2-Hydroxycarbonsäureamide sind die Amide der Äpfelsäure, Glykolsäure, Isozitronensäure Mandelsäure, Milchsäure, Tartronsäure, Weinsäure und Zitronensäure. Bevorzugt sind Glykolsäureamid und Milchsäureamid, am meisten bevorzugt ist Milchsäureamid.

Die Komplexe aus einem mehrwertigen Metallsalz der allgemeinen Formel (I) und einem 2-Hydroxycarbonsäureamid können durch Umsetzung eines mehrwertigen Metallsalzes der allgemeinen Formel (I) mit einem 2-Hydroxycarbonsäureamid hergestellt werden.

Der Komplex aus einem mehrwertigen Metallsalz der allgemeinen Formel (I) und einem 2-Hydroxycarbonsäureamid enthält vorzugsweise von 0,1 bis 3 mol, besonders bevorzugt von 0,3 bis 2 mol, ganz besonders bevorzugt von 0,5 bis 1,5 mol, des 2-Hydroxycarbonsäureamids, jeweils bezogen auf das mehrwertige Metallkation.

Zur Beschichtung eingesetzt werden vorzugsweise 0,001 bis 5 Gew.-%, besonders bevorzugt 0,01 bis 2 Gew.-%, ganz besonders bevorzugt 0,1 bis 1 Gew.-%, des Komplexes aus einem mehrwertigen Metallsalz und einem 2-Hydroxycarbonsäureamid.

In einer weiteren Ausführungsform wird der wässrigen Lösung oder Dispersion des mindestens einen Komplexes aus einem mehrwertigen Metallsalz der allgemeinen Formel (I) und einem 2-Hydroxycarbonsäureamid vor, während, oder nach dessen Synthese mindestens ein Oberflächennachvernetzer, vorzugsweise aus der Gruppe Ethylenglykol, Propylenglykol, 1,3-Propandiol, 1,4-Butandiol, Glyzerin, N-(2-Hydroxyethyl)-2-oxazolidon, 2-Oxazolidon, Ethylenkarbonat und Propylenkarbonat zugesetzt. Bezüglich der Mengen für die Zugabemengen gelten die Beschränkungen zur Oberflächennachvernetzung wie unten angegeben.

Die so hergestellte Lösung wird direkt oder weiter verdünnt eingesetzt. Ein besonderer Vorteil dieser Ausführungsform ist eine erhöhte Lagerstabilität der so hergestellten Lösungen.

Die wässrige Lösung des mindestens einen Komplexes aus einem mehrwertigen Metallsalz der allgemeinen Formel (I) und einem 2-Hydroxycarbonsäureamid stellt in der Regel eine echte Lösung oder eine kolloidale Lösung, manchmal aber auch eine Suspension dar.

Die wasserabsorbierenden Polymerpartikel sind üblicherweise wasserunlöslich.

Die Monomeren i) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere i) sind beispielsweise ethylenisch ungesättigte Carbonsäuren, wie Acrylsäure, Methacrylsäure und Itaconsäure. Besonders bevorzugte Monomere sind Acrylsäure und Methacrylsäure. Ganz besonders bevorzugt ist Acrylsäure.

Weitere geeignete Monomere i) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren i) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren i) beträgt vorzugsweise mindestens 50 mol-%, besonders bevorzugt mindestens 90 mol-%, ganz besonders bevorzugt mindestens 95 mol-%.

Die Monomere i) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer ii) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch mehrwertige Metallsalze, die mit mindestens zwei Säuregruppen des Monomeren a) koordinative Bindungen ausbilden können, als Vernetzer ii) geeignet.

Vernetzer ii) sind vorzugsweise Verbindungen mit mindestens zwei polymerisierbaren Gruppen, die in das Polymernetzwerk radikalisch einpolymerisiert werden können. Geeignete Vernetzer ii) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer ii) sind insbesondere N,N'-Methylenbisacrylamid und N,N'-Methylenbismethacrylamid, Ester ungesättigter Mono- oder Polycarbonsäuren von Polyolen, wie Diacrylate oder Triacrylate, beispielsweise Butandioldiacrylat, Ethylenglykoldiacrylat und Trimethylolpropantriacrylat, und Allylverbindungen, wie Allylacrylat, Allylmethacrylat, Triallylcyanurat, Maleinsäurediallylester, Polyallylester, Tetraallyloxyethan, Triallylamin, Tetraallylethylendiamin, Allylester der Phosphorsäure, sowie Vinylphosphonsäurederivate, wie sie beispielsweise in EP 0 343 427 A1 beschrieben sind. Weiterhin geeignete Vernetzer ii) sind Pentaerythritoldi-, Pentaerythritoltri- und Pentaerythritoltetraallylether, Polyethylenglykoldiallylether, Ethylenglykoldiallylether, Glyzerindi- und triallylether, Polyallylether auf Basis Sorbitol, sowie ethoxylierte Varianten davon. Im erfindungsgemäßen Verfahren einsetzbar sind Diacrylate und Dimethacrylate von Polyethylenglykolen, wobei das eingesetzte Polyethylenglykol ein Molekulargewicht zwischen 300 und 1000 aufweist.

Besonders vorteilhafte Vernetzer ii) sind jedoch Di- und Triacrylate des 3- bis 15-fach ethoxylierten Glyzerins, des 3- bis 15-fach ethoxylierten Trimethylolpropans, insbesondere Di- und Triacrylate des 3-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach bis 25-fach ethoxylierten Glyzerins, Trimethylolethans oder Trimethylolpropans, sowie des 40-fach ethoxylierten Glyzerins, Trimethylolethans oder Trimethylolpropans.

Ganz besonders bevorzugte Vernetzer ii) sind die mit Acrylsäure oder Methacrylsäure zu Di- oder Triacrylaten bzw. Di- oder Trimethacrylaten veresterten mehrfach ethoxylierten und/oder propoxylierten Glyzerine wie sie beispielsweise in DE 103 19 462 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 ppm) in den wasserabsorbierenden Polymerpartikeln aus und die wässrigen Extrakte der damit hergestellten gequollenen wasserabsorbierenden Polymerpartikel weisen eine fast unveränderte Oberflächenspannung (typischerweise mindestens 0,068 N/m bei 23°C) im Vergleich zu Wasser gleicher Temperatur auf.

Die Menge an Vernetzer ii) beträgt vorzugsweise 0,05 bis 2,5 Gew.-%, besonders bevorzugt 0,1 bis 1 Gew.-%, ganz besonders bevorzugt 0,3 bis 0,6 Gew.-%, jeweils bezogen auf Monomer i). Mit steigendem Vernetzergehalt sinkt die Zentrifugenretentionskapazität (CRC) und die Absorption unter einem Druck von 21,0 g/$cm^2$ (AUL0.3psi) durchläuft ein Maximum.

Mit den Monomeren i) copolymerisierbare ethylenisch ungesättigte Monomere iii) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere iv) können Polyvinylalkohol, Polyvinylamin, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer i), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung oder -suspension vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung oder-suspension vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Der Monomerlösung oder-suspension bzw. deren Rohstoffen können optional alle bekannten Chelatbildner zur besseren Kontrolle der Polymerisationsreaktion hinzugefügt werden. Geeignete Chelatbildner sind beispielsweise

Phosphorsäure, Diphosphorsäure, Triphosphorsäure, Polyphosphorsäure, Zitronensäure, Weinsäure, sowie deren Salze.

Weiterhin geeignet sind beispielsweise Iminodiessigsäure, Hydroxyethyliminodiessig-säure, Nitrilotriessigsäure, Nitrilotripropionsäure, Ethylendiamintetraessigsäure, Diethylentriaminpentaessigsäure, Triethylentetraaminhexaessigsäure, N,N-Bis(2-Hydroxyethyl)glycin und trans-1,2-diaminocyclohexantetraessigsäure, sowie deren Salze. Die Einsatzmenge beträgt üblicherweise 1 bis 30.000 ppm bezogen auf die Monomere i), vorzugsweise 10 bis 1.000 ppm, bevorzugt 20 bis 600 ppm, mehr bevorzugt 50 bis 400 ppm, am meisten bevorzugt 100 bis 300 ppm.

Die Herstellung eines geeigneten Grundpolymers sowie weitere geeignete Monomere i) werden beispielsweise in DE 199 41 423 A1, EP 0 686 650 A1, WO 2001/45758 A1 und WO 2003/104300 A1 beschrieben.

Die Umsetzung wird vorzugsweise in einem Kneter, wie in WO 2001/038402 A1 beschrieben, oder auf einem Bandreaktor, wie in EP 0 955 086 A1 beschrieben, durchgeführt. Vorteilhaft sind aber auch die Herstellung nach dem Verfahren der umgekehrten Suspensionspolymerisation oder der Tropfenpolymerisation. In beiden Verfahren werden abgerundete Grundpolymerpartikel erhalten, oft sogar mit sphärischer Morphologie.

Die Morphologie der Grundpolymerpartikel ist beliebig wählbar, beispielsweise können irreguläre scherbenförmige Partikel mit glatten Oberflächen, irreguläre Partikel mit rauen Oberflächen, Partikelaggregate, abgerundete Partikel, oder kugelförmige Partikel eingesetzt werden.

Die Polymerisation wird dabei vorteilhaft durch thermische und/oder Redoxinitiatorsysteme bewirkt. Als thermische Initiatoren geeignet sind Azoinitiatoren, Peroxodisulfate, Peroxodiphosphate und Hydroperoxide. Peroxoverbindungen, wie Wasserstoffperoxid, tert.-Butylhydroperoxid, Ammoniumpersulfat, Kaliumpersulfat und Natriumpersulfat, werden bevorzugt auch als mindestens eine Initiatorkomponente in Redoxinitiatorsystemen eingesetzt. Peroxid kann beispielsweise aber auch in-situ durch Reduktion des vorhandenen Sauerstoffs mittels eines Gemisches von Glukose und Glukoseoxidase oder mittels anderer enzymatischer Systeme erhalten werden.

Als Reduktionskomponente können beispielsweise Ascorbinsäure, Bisulfit, Thiosulfat, 2-Hydroxy-2-sulfonatoessigsäure, 2-Hydroxy-2-sulfinatoessigsäure, bzw. deren Salze, Polyamine, beispielsweise N,N,N',N'-Tetramethylethylendiamin, verwendet werden.

Die Säuregruppen der erhaltenen Polymergele sind vorzugsweise zu größer 45 mol-%, bevorzugt zu größer 55 mol-%, besonders bevorzugt zu größer 65 mol-%, ganz besonders bevorzugt zu größer 68 mol-%, und vorzugsweise zu höchstens 80 mol-%, bevorzugt zu höchstens 76 mol-%, besonders bevorzugt zu höchstens 74 mol-%, ganz besonders bevorzugt zu höchstens 72 mol-%, neutralisiert, wobei die üblichen Neutralisationsmittel verwendet werden können, beispielsweise Ammoniak, Amine, wie Ethanolamin, Diethanolamin, Triethanolamin oder Dimethylaminoethanolamin, vorzugsweise Alkalimetallhydroxide, Alkalimetalloxide, Alkalimetallkarbonate oder Alkalimetallhydrogenkarbonate sowie deren Mischungen, wobei Natrium und Kalium als Alkalimetalle besonders bevorzugt sind, ganz besonders bevorzugt jedoch Natriumhydroxid, Natriumkarbonat oder Natriumhydrogenkarbonat, sowie deren Mischungen. Optional können auch wasserlösliche Alkalisilikate zumindest zur teilweisen Neutralisation und zur Erhöhung der Gelfestigkeit eingesetzt werden. Üblicherweise wird die Neutralisation durch Einmischung des Neutralisationsmittels als wässrige Lösung oder bevorzugt auch als Feststoff erreicht.

Die Neutralisation kann nach der Polymerisation auf der Stufe des Polymergels durchgeführt werden. Es ist aber auch möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Die Monomerlösung kann durch Einmischen des Neutralisationsmittels neutralisiert werden, entweder auf einen vorbestimmten Vorneutralisationgrad mit anschließender Nachneutralisation auf den Endwert nach oder während der Polymerisationsreaktion, oder die Monomerlösung wird durch Einmischen des Neutralisationsmittels vor der Polymerisation direkt auf den Endwert eingestellt. Das Polymergel kann mechanisch zerkleinert werden, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Bei zu geringem Neutralisationsgrad kommt es bei der anschließenden Trocknung sowie während der anschließenden Oberflächennachvernetzung des Grundpolymeren zu unerwünschten thermischen Vernetzungseffekten, welche die Zentrifugenretentionskapazität (CRC) der wasserabsorbierenden Polymerpartikel stark verringern können, bis hin zur Unbrauchbarkeit.

Bei zu hohem Neutralisationsgrad kommt es jedoch zu einer weniger effizienten Oberflächennachvernetzung, was zu einer verringerten Flüssigkeitsweiterleitung (SFC) der wasserabsorbierenden Polymerpartikel führt.

Ein optimales Ergebnis erhält man hingegen wenn man den Neutralisationsgrad des Grundpolymeren so einstellt, dass man eine effiziente Oberflächennachvernetzung erreicht und somit eine hohe Flüssigkeitsweiterleitung (SFC), wobei man gleichzeitig aber noch so weit neutralisiert, dass man das Polymergel bei der Herstellung in einem gewöhnlichen Bandtrockner oder anderen im technischen Maßstab üblichen Trocknungsapparaturen ohne Verlust an Zentrifugenretentionskapazität (CRC) trocknen kann.

Vor der Trocknung kann das Polymergel noch mechanisch nachbearbeitet werden um verbliebene Klumpen

zu zerkleinern oder die Größe und Struktur der Gelpartikel zu homogenisieren. Dazu können rührende, knetende, formende, scherende und schneidende Werkzeuge eingesetzt werden. Eine zu starke Scherbeanspruchung kann das Polymergel jedoch schädigen. In der Regel führt eine milde mechanische Nachbearbeitung zu einem verbesserten Trocknungsergebnis, da die regelmäßigeren Gelteilchen gleichmäßiger trocknen und zu weniger Blasen und Klumpen neigen.

Das neutralisierte Polymergel wird dann mit einem Band-, Wirbelschicht-, Schacht- oder Walzentrockner getrocknet bis der Restfeuchtegehalt vorzugsweise unter 10 Gew.-%, insbesondere unter 5 Gew.-% liegt, wobei der Restfeuchtegehalt gemäß der von der EDANA empfohlenen Testmethode Nr. WSP 230.2-05 "Moisture content" bestimmt wird. Das getrocknete Polymergel wird hiernach gemahlen und gesiebt, wobei zur Mahlung üblicherweise Walzenstühle, Stiftmühlen oder Schwingmühlen eingesetzt werden können, wobei Siebe mit zur Herstellung der wasserabsorbierenden Polymerpartikel notwendigen Maschenweiten verwendet werden.

Die Grundpolymere werden anschließend oberflächennachvernetzt. Hierzu geeignete Oberflächennachvernetzer v) sind Verbindungen, die mindestens zwei Gruppen enthalten, die mit den Carboxylatgruppen der Polymere kovalente Bindungen bilden können. Geeignete Verbindungen sind beispielsweise Alkoxysilylverbindungen, Polyaziridine, Polyamine, Polyamidoamine, Di- oder Polyglycidylverbindungen, wie in EP 0 083 022 A2, EP 0 543 303 A1 und EP 0 937 736 A2 beschrieben, mehrwertige Alkohole, wie in DE 33 14 019 A1, DE 35 23 617 A1 und EP 0 450 922 A2 beschrieben, oder ß-Hydroxyaikyiamide, wie in DE 102 04 938 A1 und US 6,239,230 beschrieben. Geeignet sind ferner Verbindungen mit gemischter Funktionalität, wie Glycidol, 3-Ethyl-3-oxetanmethanol (Trimethylolpropanoxetan), wie in EP 1 199 327 A1 beschrieben, Aminoethanol, Diethanolamin, Triethanolamin oder Verbindungen, die nach der ersten Reaktion eine weitere Funktionalität ausbilden, wie Ethylenoxid, Propylenoxid, Isobutylenoxid, Aziridin, Azetidin oder Oxetan.

Des weiteren sind in DE 40 20 780 C1 zyklische Karbonate, in DE 198 07 502 A1 2-Oxazolidon und dessen Derivate, wie N-(2-Hydroxyethyl)-2-oxazolldon, in DE 198 07 992 C1 Bis- und Poly-2-oxazolidone, in DE 198 54 573 A1 2-Oxotetrahydro-1,3-oxazin und dessen Derivate, in DE 198 54 574 A1 N-Acyl-2-Oxazolidone, in DE-102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer v) beschrieben.

Die Oberflächennachvernetzung wird üblicherweise so durchgeführt, dass eine Lösung des Oberflächennachvernetzers auf die trockenen Grundpolymerpartikel aufgesprüht wird. Im Anschluss an das Aufsprühen wird thermisch oberflächennachvernetzt, wobei sowohl vor als auch während der Oberflächennachvernetzungsreaktion Trocknung stattfinden kann.

Bevorzugte Oberflächennachvernetzer v) sind Amidacetale oder Carbaminsäureester der allgemeinen Formel (II)

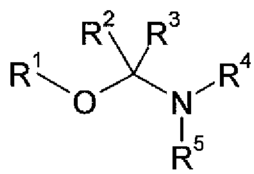

(II),

worin

- $R^1$ $C_1$-$C_{12}$-Alkyl, $C_2$-$C_{12}$-Hydroxyalkyl, $C_2$-$C_{12}$-Alkenyl oder $C_6$-$C_{12}$-Aryl,
- $R^2$ Z oder $OR^6$
- $R^3$ Wasserstoff, $C_1$-$C_{12}$-Alkyl, $C_2$-$C_{12}$-Hydroxyalkyl, $C_2$-$C_{12}$-Alkenyl oder $C_6$-$C_{12}$-Aryl, oder Z,
- $R^4$ $C_1$-$C_{12}$-Alkyl, $C_2$-$C_{12}$-Hydroxyalkyl, $C_2$-$C_{12}$-Alkenyl oder $C_6$-$C_{12}$-Aryl,
- $R^5$ Wasserstoff, $C_1$-$C_{12}$-Alkyl, $C_2$-$C_{12}$-Hydroxyalkyl, $C_2$-$C_{12}$-Alkenyl, $C_1$-$C_{12}$-Acyl oder $C_6$-$C_{12}$-Aryl,
- $R^6$ $C_1$-$C_{12}$-Alkyl, $C_2$-$C_{12}$-Hydroxyalkyl, $C_2$-$C_{12}$-Alkenyl oder $C_6$-$C_{12}$-Aryl und
- Z ein für die Reste $R^2$ und $R^3$ gemeinsamer Carbonyl-Sauerstoff

bedeuten, wobei $R^1$ und $R^4$ und/oder $R^5$ und $R^6$ ein verbrücktes $C_2$- bis $C_6$-Alkandiyl sein können, und wobei die obengenannte Reste $R^1$ bis $R^6$ noch insgesamt über ein bis zwei freie Valenzen verfügen können und mit diesen freien Valenzen mit mindestens einem geeigneten Grundkörper verbunden sein können,
oder mehrwertige Alkohole, wobei der mehrwertige Alkohol vorzugsweise ein Molekulargewicht von weniger als 100 g/mol, bevorzugt von weniger als 90 g/mol, besonders bevorzugt von weniger als 80 g/mol, ganz besonders bevorzugt von weniger als 70 g/mol, pro Hydroxylgruppe sowie keine vicinalen geminalen, sekundären oder tertiären Hydroxylgruppen aufweist, und mehrwertige Alkohole entweder Diole der allgemeinen Formel (IIIa)

HO-$R^7$-OH (IIIa),

worin $R^7$ entweder einen unverzweigten Dialkylrest der Formel $-(CH_2)_p-$ , wobei p eine ganze Zahl von 2 bis 20, bevorzugt 3 bis 12 ist, bedeutet und beide Hydroxylgruppen endständig sind, oder $R^7$ einen unverzweigten, verzweigten oder zyklischen Dialkylrest bedeutet, oder Polyole der allgemeinen Formel (IIIb)

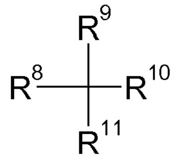

(IIIb),

worin die Reste $R^8$, $R^9$, $R^{10}$, $R^{11}$ unabhängig voneinander Wasserstoff, Hydroxyl, Hydroxymethyl, Hydroxyethyloxymethyl, 1-Hydroxyprop-2-yloxymethyl, 2-Hydroxypropyloxymethyl, Methyl, Ethyl, n-Propyl, Isopropyl, n-Butyl, n-Pentyl, n-Hexyl, 1,2-Dihydroxyethyl, 2-Hydroxyethyl, 3-Hydroxypropyl oder 4-Hydroxybutyl bedeuten und insgesamt 2, 3, oder 4, bevorzugt 2 oder 3, Hydroxylgruppen vorhanden sind, und nicht mehr als einer der Reste $R^8$, $R^9$, $R^{10}$, oder $R^{11}$ gleich Hydroxyl bedeutet, sind,
oder zyklische Karbonate der allgemeinen Formel (IV)

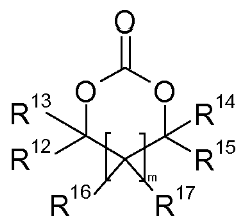

(IV),

worin $R^{12}$, $R^{13}$, $R^{14}$, $R^{15}$ $R^{16}$ und $R^{17}$ unabhängig voneinander Wasserstoff, Methyl, Ethyl, n-Propyl, Isopropyl, n-Butyl, sek.-Butyl oder Isobutyl, und m entweder 0 oder 1 ist,
oder Bisoxazoline der allgemeinen Formel (V)

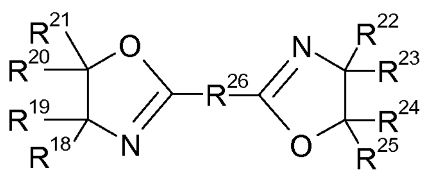

(V),

worin $R^{18}$, $R^{19}$, $R^{20}$, $R^{21}$, $R^{22}$, $R^{23}$, $R^{24}$ und $R^{25}$ unabhängig voneinander Wasserstoff, Methyl, Ethyl, n-Propyl, Isopropyl, n-Butyl, sek.-Butyl oder Isobutyl, und $R^{26}$ eine Einfachbindung, einen linearen, verzweigten oder zyklischen $C_1$-$C_{12}$-Dialkylrest, oder einen Polyalkoxydiylrest darstellt, welcher aus ein bis zehn Ethylenoxid- und/oder Propylenoxideinheiten aufgebaut ist, wie sie beispielsweise Polyglykoldicarbonsäuren aufweisen.

Die bevorzugten Oberflächennachvernetzer v) sind außerordentlich selektiv. Neben- und Folgereaktionen, die zu flüchtigen und damit übelriechenden Verbindungen führen sind minimiert. Die mit den bevorzugten Oberflächennachvernetzern v) hergestellten wasserabsorbierenden Polymerpartikel sind daher auch im angefeuchteten Zustand geruchsneutral.

Mehrwertige Alkohole als Oberflächennachvernetzer v) benötigen aufgrund ihrer geringen Reaktivität hohe Oberflächennachvernetzungstemperaturen. Alkohole, die vincinale, geminale, sekundäre und tertiäre Hydroxylgruppen aufweisen, bilden dabei im Hygienebereich unerwünschte Nebenprodukte, die zu unangenehmen Gerüchen und/oder Verfärbungen des betreffenden Hygieneartikels während der Herstellung oder des Gebrauchs führen.

Bevorzugte Oberflächennachvernetzer v) der allgemeinen Formel (II) sind 2-Oxazolidone, wie 2-Oxazolidon und N-Hydroxyethyl-2-oxazolidon.

Bevorzugte Oberflächennachvernetzer v) der allgemeinen Formel (IIIa) sind 1,3-Propandiol, 1,4-Butandiol, 1,5-Pentandiol, 1,6-Hexandiol und 1,7-Heptandiol. Weitere Beispiele für Oberflächennachvernetzer der Formel (IIIa) sind 1,3-Butandiol, 1,8-Oktandiol, 1,9-Nonandiol und 1,10-Dekandiol.

Die Diole der allgemeinen Formel (IIIa) sind vorzugsweise wasserlöslich, wobei diese Diole sich bei 23°C zu

mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche Oberflächennachvernetzer die bei 25°C flüssig sind.

Bevorzugte Oberflächennachvernetzer v) der allgemeinen Formel (IIIb) sind Butan-1,2,3-triol, Butan-1,2,4-triol, Glyzerin, Trimethylolpropan, Trimethylolethan, Pentaerythrit, 1- bis 3-fachethoxyliertes Glyzerin, Trimethylolethan oder Trimethylolpropan und 1- bis 3-fach-propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin und Trimethylolpropan.

Bevorzugte mehrwertige Alkohole der allgemeinen Formeln (IIIa) und (IIIb) weisen bei 23°C eine Viskosität von weniger als 3.000 mPas, vorzugsweise weniger als 1.500 mPas, bevorzugt weniger als 1.000 mPas, besonders bevorzugt weniger als 500 mPas, ganz besonders bevorzugt weniger als 300 mPas, auf.

Besonders bevorzugte Oberflächennachvernetzer v) der allgemeinen Formel (IV) sind Ethylenkarbonat und Propylenkarbonat.

Ein besonders bevorzugter Oberflächennachvernetzer v) der allgemeinen Formel (V) ist 2,2'-Bis(2-oxazolin).

Der mindestens eine Oberflächennachvernetzer v) wird typischerweise in einer Menge von höchstens 0,3 Gew.-%, vorzugsweise von höchstens 0,15 Gew.-%, besonders bevorzugt von 0,001 bis 0,095 Gew.-%, jeweils bezogen auf das Grundpolymer, als wässrige Lösung eingesetzt.

Es kann ein einzelner Oberflächennachvernetzer v) aus der obigen Auswahl verwendet werden oder beliebige Gemische verschiedener Oberflächennachvernetzer.

Die wässrige Oberflächennachvernetzerlösung kann neben dem mindestens einen Oberflächennachvernetzer v) typischerweise noch ein Cosolvens enthalten.

Technisch gut geeignete Cosolventien sind $C_1$- bis $C_6$-Alkohole, wie Methanol, Ethanol, n-Propanol, Isopropanol, n-Butanol, sek.-Butanol, tert.-Butanol oder 2-Methyl-1-Propanol, $C_2$-bis $C_5$-Diole, wie Ethylenglykol, Propylenglykol oder 1,4-Butandiol, Ketone, wie Aceton, oder Carbonsäureester, wie Essigsäureethylester. Nachteilig an vielen dieser Cosolventien ist, dass sie typische Eigengerüche aufweisen.

Das Cosolvens selbst ist unter den Reaktionsbedingungen idealerweise kein Oberflächennachvernetzer. Es kann jedoch im Grenzfall und abhängig von Verweilzeit und Temperatur dazu kommen, dass das Cosolvens teilweise zur Oberflächennachvernetzung beiträgt. Dies ist insbesondere dann der Fall, wenn der Oberflächennachvernetzer v) relativ träge ist und daher auch selbst sein Cosolvens bilden kann, wie beispielsweise bei Einsatz zyklischer Karbonate der allgemeinen Formel (IV), Diole der allgemeinen Formel (IIIa) oder Polyole der allgemeinen Formel (IIIb). Solche Oberflächennachvernetzer v) können im Gemisch mit reaktiveren Oberflächennachvernetzern v) auch in der Funktion als Cosolvens eingesetzt werden, da die eigentliche Oberflächennachvernetzungsreaktion dann bei niedrigeren Temperaturen und/oder kürzeren Verweilzeiten als in Abwesenheit des reaktiveren Oberflächennachvernetzers v) durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die Diole der allgemeinen Formel (IIIa), die Polyole der allgemeinen Formel (IIIb), sowie die zyklischen Karbonate der allgemeinen Formel (IV) auch als Cosolventien. Diese Funktion erfüllen sie in Gegenwart eines reaktiven Oberflächennachvernetzers v) der allgemeinen Formel (II) und/oder (V) und/oder eines Di- oder Triyglycidylvernetzers. Bevorzugte Cosolventien im erfindungsgemäßen Verfahren sind jedoch insbesondere die Diole der allgemeinen Formel (IIIa).

Weitere im erfindungsgemäßen Verfahren besonders bevorzugte Cosolventien sind die Polyole der allgemeinen Formel (IIIb). Insbesondere bevorzugt sind hiervon die 2- bis 3-fach alkoxylierten Polyole. Besonders geeignet als Cosolventien sind aber auch 3- bis 15-fach, ganz besonders 5- bis 10-fach ethoxylierte Polyole auf der Basis von Glyzerin, Trimethylolpropan, Trimethylolethan oder Pentaerythrit. Besonders gut geeignet ist 7-fach ethoxyliertes Trimethylolpropan.

Besonders bevorzugte Kombinationen aus wenig reaktivem Oberflächennachvernetzer v) als Cosolvens und reaktivem Oberflächennachvernetzter v) sind Kombinationen von bevorzugten mehrwertigen Alkoholen, Diolen der allgemeinen Formel (IIIa) und Polyolen der allgemeinen Formel (IIIb), mit Amidacetalen oder Carbaminsäureestern der allgemeinen Formel (II).

Ganz besonders bevorzugte Kombinationen sind 2-Oxazolidon/1,3-Propandiol, 2-Oxazolidon/Propylenglykol, N-(2-Hydroxyethyl)-2-oxazolidon/1,3-Propandiol sowie N-(2-Hydroxyethyl)-2-oxazolidon/Propylenglykol.

Weitere bevorzugte Kombinationen sind Propylenglykol/1,4-Butandiol, Propylenglykol/1,3-Propandiol, 1,3-Propandiol/1,4-Butandiol, gelöst in Wasser und/oder Isopropanol als nichtreaktivem Lösemittel.

Weitere bevorzugte Oberflächennachvernetzermischungen sind Ethylenkarbonat/Wasser und 1,3-Propandiol/Wasser. Diese können gegebenenfalls mit Isopropanol gemischt eingesetzt werden.

Häufig beträgt die Konzentration des Cosolvens in der wässrigen Oberflächennachvernetzerlösung, von 15 bis 50 Gew.-%, vorzugsweise von 15 bis 40 Gew.-%, besonders bevorzugt von 20 bis 35 Gew.-%, bezogen auf die Lösung. Bei Cosolventien, die mit Wasser nur begrenzt mischbar sind, wird man vorteilhaft die wässrige Oberflächennachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der mindestens eine Oberflächennachvernetzer v) wird dann nur als Lösung in Wasser angewandt, gegebenenfalls unter Zusatz eines Deagglomerationshilfsmittels.

Die Konzentration des mindestens einen Oberflächennachvernetzers v) in der wässrigen Lösung, beträgt beispielsweise 1 bis 20 Gew.-%, vorzugsweise 1,5 bis 10 Gew.-%, besonders bevorzugt 2 bis 5 Gew.-%, bezogen auf die Lösung.

Die Gesamtmenge der Oberflächennachvernetzerlösung bezogen auf Grundpolymer beträgt üblicherweise von 0,3 bis 15 Gew.-%, vorzugsweise von 2 bis 6 Gew.-%.

In einer bevorzugten Ausführungsform wird dem Grundpolymer ein Tensid als Deagglomerationshilfsmittel, beispielsweise Sorbitanmonoester, wie Sorbitanmonococoat und Sorbitanmonolaurat, oder ethoxylierte Varianten davon zugesetzt. Weitere sehr geeignete Deagglomerationshilfsmittel stellen die ethoxylierten und alkoxylierten Derivate des 2-Propylheptanols dar, die unter den Handelsmarken Lutensol XL® und Lutensol XP® vertrieben werden (BASF SE, Ludwigshafen, DE). Das Deagglomerationshilfsmittel kann getrennt dosiert oder der Oberflächennachvernetzerlösung zugesetzt werden. Vorzugsweise wird das Deagglomerationshilfsmittel der Oberflächennachvernetzerlösung zugesetzt.

Die Einsatzmenge des Deagglomerationshilfsmittels bezogen auf Grundpolymer beträgt beispielsweise bis 0,01 Gew.-%, vorzugsweise bis 0,005 Gew.-%, besonders bevorzugt bis 0,002 Gew.-%. Vorzugsweise wird das Deagglomerationshilfsmittel so dosiert, dass die Oberflächenspannung eines wässrigen Extrakts des gequollenen Grundpolymers und/oder der gequollenen oberflächennachvernetzten wasserabsorbierenden Polymerpartikel bei 23°C üblicherweise mindestens 0,05 N/m, vorzugsweise mindestens 0,055 N/m, bevorzugt mindestens 0,06 N/m, besonders bevorzugt mindestens 0,065 N/m, ganz besonders bevorzugt 0,068 N/m, beträgt.

Der mindestens eine Komplex aus einem mehrwertigem Metallsalz der allgemeinen Formel (I) und einem 2-Hydroxycarbonsäureamid kann als wässrige Lösung vor, während, gemeinsam oder nach dem Aufbringen der Oberflächennachvernetzerlösung aufgesprüht werden. Es kann auch nach Abschluss der thermischen Oberflächennachvernetzung aufgebracht werden. Bevorzugt ist jedoch der Auftrag während dem Aufbringen der Oberflächennachvernetzerlösung aus mindestens zwei parallelen Düsen. Am meisten bevorzugt ist der Auftrag zusammen mit der Oberflächennachvernetzerlösung aus einer gemeinsamen Lösung des Oberflächennachvernetzers und des mindestens einen Komplexes aus einem mehrwertigen Metallsalz der allgemeinen Formel (I) und einem 2-Hydroxycarbonsäureamid. Dazu können eine oder mehrere Düsen zum Aufsprühen der Lösung verwendet werden.

Das im erfindungsgemäßen Verfahren eingesetzte Grundpolymer weist typischerweise einen Restfeuchtegehalt nach der Trocknung und vor Aufbringen der Oberflächennachvernetzerlösung von unter 10 Gew.-%, bevorzugt unter 5 Gew.-%, auf. Optional kann dieser Feuchtegehalt aber auch, beispielsweise durch Aufbringen von Wasser in einem vorgeschalteten Sprühmischer, auf bis zu 75 Gew.-% erhöht werden. Der Feuchtegehalt wird gemäß der von der EDANA empfohlenen Testmethode Nr. WSP 230.2-05 "Moisture content" bestimmt. Eine solche Erhöhung des Feuchtegehalts führt zu einer leichten Vorquellung des Grundpolymers und verbessert die Verteilung des Oberflächennachvernetzers auf der Oberfläche sowie die Durchdringung der Partikel.

Die im erfindungsgemäßen Verfahren einsetzbaren Sprühdüsen unterliegen keiner Beschränkung. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP 0 534 228 A1 und der EP 1 191 051 A1 beschrieben.

Im Anschluss an das Aufsprühen wird thermisch oberflächennachvernetzt, wobei sowohl vor, während oder nach der Oberflächennachvernetzungsreaktion Trocknung stattfinden kann.

Das Aufsprühen der Oberflächennachvernetzerlösung wird vorzugsweise in Mischern mit bewegten Mischwerkzeugen, wie Schneckenmischer, Paddelmischer, Scheibenmischer, Pflugscharmischer und Schaufelmischer, durchgeführt. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und ara®-Trockner. Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke.

Besonders bevorzugt wird die Oberflächennachvernetzerlösung in einem Hochgeschwindigkeitsmischer, beispielsweise vom Typ Schugi-Flexomix® oder Turbolizer®, auf das Grundpolymer aufgebracht und in einem Reaktionstrockner, beispielsweise vom Typ Nara-Paddle-Dryer® oder einem Scheibentrockner, thermisch oberflächennachvernetzt. Das eingesetzte Grundpolymer kann aus vorhergehenden Prozessschritten noch eine Temperatur von 10 bis 120°C aufweisen, die Oberflächennachvernetzerlösung kann eine Temperatur von 0 bis 150 °C aufweisen. Insbesondere kann die Oberflächennachvernetzerlösung zur Verminderung der Viskosität erwärmt werden. Bevorzugt zur Oberflächennachvernetzung und Trocknung ist dabei der Temperaturbereich von 30 bis 220°C, insbesondere 140 bis 210°C, besonders bevorzugt 160 bis 190°C. Die bevorzugte Verweilzeit bei dieser Temperatur im Reaktionsmischer oder Trockner beträgt unter 120 Minuten, mehr bevorzugt unter 80 Minuten, besonders bevorzugt unter 50 Minuten, am meisten bevorzugt unter 30 Minuten.

Der Oberflächennachvernetzungstrockner wird während der Trocknungs- und Oberflächennachvernetzungsreaktion mit Luft oder einem Inertgas gespült, um die Dämpfe zu entfernen. Zur Unterstützung der Trocknung werden der Trockner und die angeschlossenen Aggregate möglichst vollständig beheizt.

Selbstverständlich können mit den Dämpfen abgeführte Cosolventien außerhalb des Reaktionstrockners wieder kondensiert und gegebenenfalls destillativ aufgetrennt und rezyklisiert werden.

In einer bevorzugten Ausführungsform wird die Oberflächennachvernetzungsreaktion und die Trocknung in Abwesenheit oxidierender Gase, insbesondere Sauerstoff durchgeführt, wobei der Anteil an oxidierendem Gas in der die wasserabsorbierenden Polymerpartikel überlagernden Atmosphäre weniger als 10 Vol.-%, vorzugsweise weniger als 1 Vol.-%, bevorzugt weniger als 0,1 Vol.-%, besonders bevorzugt weniger als 0,01 Vol.-%, ganz besonders bevorzugt weniger als 0,001 Vol.-%, beträgt.

Nach Abschluss der Reaktionstrocknung werden die getrockneten wasserabsorbierenden Polymerpartikel abgekühlt. Vorzugsweise überführt man dazu die warmen und trockenen Polymerpartikel im kontinuierlichen Betrieb in einen nachgeschalteten Kühler. Dieser kann beispielsweise ein Scheibenkühler, ein Schaufelkühler, ein Wirbelschichtkühler, oder ein Schneckenkühler sein. Gekühlt wird dabei über die Wände und gegebenenfalls die Rührorgane des Kühlers, welche von einem geeigneten Kühlmedium wie beispielsweise Warm- oder Kaltwasser durchströmt werden. Zweckmäßig können im Kühler Wasser oder wässrige Lösungen von Additiven aufgesprüht werden; dadurch erhöht sich die Effizienz der Kühlung (partielle Wasserverdampfung). Der erhöhte Restfeuchtegehalt verringert den Staubgehalt des Produkts.

Geeignete Additive sind beispielsweise pyrogene Kieselsäuren und Tenside, die das Verbacken der Polymerpartikel bei Wasserzugabe verhindern. Optional kann aber auch eine wässrige Lösung des mindestens einen Komplexes aus einem mehrwertigen Metallsalz der allgemeinen Formel (I) und einem 2-Hydroxycarbonsäureamid hier aufgebracht werden.

Weitere besonders geeignete Additive sind farbstabilisierende Zusätze wie beispielsweise Natriumbisulfit, Natriumhypophosphit, Phosphatsalze, 2-Hydroxy-2-sulfonatoessigsäure oder deren Salze, 2-Hydroxy-2-sulfinatoessigsäure oder deren Salze, 1-Hydroxyethyliden-1,1-diphosphonsäure oder deren Salze, Glyoxylsäure oder deren Salze, insbesondere die Kalzium und Strontiumsalze.

Optional kann jedoch im Kühler auch nur gekühlt werden und die Zugabe von Wasser und Additiven in einem nachgeschalteten separaten Mischer durchgeführt werden. Die Kühlung stoppt die Reaktion durch Unterschreiten der Reaktionstemperatur und die Temperatur braucht insgesamt nur so weit abgesenkt werden, dass das Produkt sich problemlos in Plastiksäcke oder in Silo-LKW abpacken lässt.

Die wasserabsorbierenden Polymerpartikel können optional mit wasserunlöslichen Metallphosphaten zusätzlich beschichtet werden, wie in der WO 2002/060983 A1 beschrieben.

Optional können alle bekannten Beschichtungen, wie filmbildende Polymere, Dendrimere, polykationische Polymere (wie Polyvinylamin, Polyethylenimin oder Polyallylamin), wasserunlösliche mehrwertige Metallsalze, wie Kalziumsulfat, oder hydrophile anorganische Partikel, wie Tonminerale, pyrogene Kieselsäure, Aluminiumoxid und Magnesiumoxid, zusätzlich aufgebracht werden. Dadurch können zusätzliche Effekte, beispielsweise eine verringerte Verbackungsneigung, verbesserte Verarbeitungseigenschaften oder eine weiter gesteigerte Flüssigkeitsweiterleitung (SFC) erreicht werden. Wenn die Additive in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche der wasserabsorbierenden Polymerpartikel aufgebracht.

Ein weiterer Gegenstand der vorliegenden Erfindung sind Hygieneartikel, enthaltend erfindungsgemäße wasserabsorbierende Polymerpartikel, vorzugsweise ultradünne Windeln, enthaltend einen absorbierenden Kern bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer wasserabsorbierender Polymerpartikel, wobei die Umhüllung des absorbierenden Kerns selbstverständlich nicht berücksichtigt ist.

Ganz besonders vorteilhaft sind die erfindungsgemäßen wasserabsorbierenden Polymerpartikel auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierender Strukturen beschriebenen Schmelzklebern und insbesondere den in der US 2003/0181115 beschriebenen

Fasern aus Schmelzklebern, an die die wasserabsorbierenden Polymerpartikel gebunden sind, eignen sich die erfindungsgemäßen wasserabsorbierenden Polymerpartikel auch zur Herstellung von vollkommen analogen Strukturen unter Verwendung von UV-vernetzbaren Schmelzklebern, welche beispielsweise als AC-Resin® (BASF SE, Ludwigshafen, DE) vertrieben werden. Diese UV-vernetzbaren Schmelzkleber haben den Vorteil bereits bei 120 bis 140°C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen wasserabsorbierenden Polymerpartikeln, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen wasserabsorbierenden Polymerpartikel mit UV-vernetzbaren Schmelzklebern ist daher besonders vorteilhaft. Geeignete UV-vernetzbare Schmelzkleber sind beispielsweise beschrieben in EP 0 377 199 A1, EP 0 445 641 A1, US 5,026,806, EP 0 655 465 A1 und EP 0 377 191 A1.

Zellulosefreie Hygieneartikel werden durch Fixierung von wasserabsorbierenden Polymerpartikeln mittels thermoplastischer Polymere, insbesondere von Schmelzklebern, auf geeigneten Vliesträgern befestigt wenn man diese thermoplastischen Polymere zu feinen Fasern ausspinnt. Solche Produkte sind in US 2004/0167486, US 2004/0071363, US 2005/0097025, US 2007/0156108, US 2008/0125735, EP 1 917 940 A2, EP 1 913 912 A1, EP 1 913 913 A2, EP 1 913 914 A1, EP 1 911 425 A2, EP 1 911 426 A2, EP 1 447 067 A1, EP 1 813 237 A2, EP 1 813 236 A2, EP 1 808 152 A2 und EP 1 447 066 A1 beschrieben. Die Herstellverfahren sind in den WO 2008/155722 A2, WO 2008/155702 A1, WO 2008/155711 A1, WO 2008/155710 A1, WO 2008/155701 A2, WO 2008/155699 A1 beschrieben. Weiterhin sind dehnbare Zellulosefreie Hygieneartikel bekannt und in den US 2006/0004336, US 2007/0135785, US 2005/0137085 ist deren Herstellung durch gleichzeitiges Faserspinnen von geeigneten thermoplastischen Polymeren und Einarbeitung von pulverförmigen wasserabsorbierenden Polymerpartikeln offenbart.

Die wasserabsorbierenden Polymerpartikel werden mit den nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Centrifuge Retention Capacity" bestimmt, jedoch wird abweichend davon für jedes Beispiel die tatsächliche Probe mit der dort angegebenen Partikelgrößenverteilung gemessen.

Absorption unter einem Druck von 49,2 g/cm$^2$ (Absorbency Under Pressure)

Die Absorption unter einem Druck von 49,2 g/cm$^2$ (AUL0.7psi) wird analog der von der EDANA empfohlenen Testmethode Nr. WSP 242.2-05 "Absorption Under Pressure" bestimmt, jedoch wird abweichend davon statt eines Druckes von 21,0 g/cm$^2$ (AUL0.3psi) ein Druck von 49,2 g/cm$^2$ (AUL0.7psi) eingestellt und für jedes Beispiel die tatsächliche Probe mit der dort angegebenen Partikelgrößenverteilung gemessen.

Gelbettpermeabilität (Gel Bed Permeability)

Die Gelbettpermeabilität (GBP) einer gequollenen Gelschicht unter Druckbelastung von 0,3 psi (2070 Pa) wird, wie in US 2005/0256757 beschrieben (Absätze [0061] und [0075]), als Gel-Bed-Permeability einer gequollenen Gelschicht aus wasserabsorbierenden Polymerpartikeln bestimmt.

Quellgeschwindigkeit (Free Swell Rate)

Zur Bestimmung der Quellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas

absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$FSR\ [g/gs] = W2/(W1xt)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Die EDANA-Testmethoden sind beispielsweise erhältlich bei der EDANA, Avenue Eugene Plasky 157, B-1030 Brüssel, Belgien.

Beispiele

Herstellung des Grundpolymers:

Beispiel 1

Durch kontinuierliches Mischen von entionisiertem Wasser, 50 gew.-%iger Natronlauge und Acrylsäure wurde eine Acrylsäure/Natriumacrylatlösung hergestellt, so dass der Neutralisationsgrad 71 mol-% entsprach. Der Feststoffgehalt der Monomerlösung betrug 40 Gew.-%.

Als mehrfach ethylenisch ungesättigter Vernetzer wurde 3-fach ethoxyliertes Glycerintriacrylat (ca. 85gew.-%ig) verwendet. Die Einsatzmenge betrug 1,5 kg Vernetzer pro t Monomerlösung.

Zur Initiierung der radikalischen Polymerisation wurden pro t Monomerlösung 1 kg einer 0,25gew.-%igen wässriger Wasserstoffperoxidlösung, 1,5 kg einer 30gew.-%igen wässrigen Natriumperoxodisulfatlösung und 1 kg einer 1gew.-%igen wässrigen Ascorbinsäurelösung eingesetzt.

Der Durchsatz der Monomerlösung betrug 18 t/h. Die Reaktionslösung hatte am Zulauf eine Temperatur von 30°C.

Die einzelnen Komponenten wurden in folgenden Mengen kontinuierlich in einen Reaktor vom Typ List Contikneter mit einem Volumen 6,3m$^3$ (LIST AG, Arisdorf, CH) dosiert:

| | |
|---|---|
| 18 t/h | Monomerlösung |
| 27 kg/h | 3-fach ethoxyliertes Glycerintriacrylat |
| 45 kg/h | Wasserstoffperoxidlösung/Natriumperoxodisulfatlösung |
| 18 kg/h | Ascorbinsäurelösung |

Zwischen dem Zugabepunkt für den Vernetzer und den Zugabestellen für die Initiatoren wurde die Monomerlösung mit Stickstoff inertisiert.

Es fand nach ca. 50% der Verweilzeit zusätzlich eine Zudosierung von aus dem Herstellungsprozess durch Mahlung und Siebung anfallendem Feinkorn (1000 kg/h) in den Reaktor statt. Die Verweilzeit der Reaktionsmischung im Reaktor betrug 15 Minuten.

Das erhaltene Polymergel wurde auf einen Bandtrockner aufgegeben. Auf dem Bandtrockner wurde das Polymergel kontinuierlich mit einem Luft/Gasgemisch umströmt und getrocknet. Die Verweilzeit im Bandtrockner betrug 37 Minuten.

Das getrocknete Polymergel wurde gemahlen und auf eine Partikelgrößenfraktion von 150 bis 850 μm abgesiebt.

Die erhaltenen wasserabsorbierenden Polymerpartikel (Grundpolymer) wiesen folgende Partikelgrößenverteilung auf:

| | |
|---|---|
| >800 μm | 2,5 Gew.-% |
| 300 bis 600 μm | 82,6 Gew.-% |
| 200 bis 300 μm | 11,0 Gew.% |
| 100 bis 200 μm | 3,7 Gew.-% |
| <100 μm | 0,2 Gew.-% |

Die erhaltenen wasserabsorbierenden Polymerpartikel (Grundpolymer) wiesen eine Zentrifugenretentionskapazität (CRC) von 38,7 g/g, Absorption unter einem Druck von 49,2 g/$cm^2$ (AUL0.7psi) von 7,3 g/g und eine Quellgeschwindigkeit (FSR) von 0,27 g/gs auf.

Herstellung der Komplexe

Beispiel 2

In einem 500 mL Vierhalsrundkolben wurden 43,0 g (552 mmol) Aluminiumhydroxid vorgelegt. Der Kolben wurde in ein auf 80°C vorgeheiztes Ölbad eingetaucht. Es wurden 250 mL Wasser hinzugegeben und mit einem Rührfisch unter Verwendung einer Magnetrührheizplatte langsam und kontinuierlich gerührt. Anschließend wurden zu der Mischung 49,1 g (552) mmol Milchsäureamid (ca. 98gew.-%ig) gegeben. Es wurden zusätzlich ein Thermometer, ein Blasenzähler und ein Rückflusskühler auf den Kolben aufgesetzt und die Mischung über Nacht bei 75°C gerührt (15 h). Die Lösung wurde anschließend abgekühlt und direkt, ohne weitere Nachbehandlung eingesetzt.

Beispiel 3

Es wurde verfahren wie unter Beispiel 2, wobei 10,6 g (136 mmol) Aluminiumhydroxid, 36,3 g (408 mmol) Milchsäureamid und 120 ml Wasser eingesetzt wurden.

Beispiel 4

Es wurde verfahren wie unter Beispiel 2, wobei 22,1 g (283 mmol) Aluminiumhydroxid, 76,5 g (850 mmol) Milchsäure und 250 ml Wasser eingesetzt wurden.

Oberflächennachvernetzung des Grundpolymers

Beispiel 5

In einem Pflugschar®-Schaufeltrockner Typ M5RMK mit 5 l Volumen (Gebr. Lödige Maschinenbau GmbH; Paderborn, Deutschland) wurden 1,2 kg Grundpolymer aus Beispiel 1 vorgelegt. Bei einer Umgebungstemperatur (23°C) wurde unter Rühren (200 U/min) innerhalb von ca. 80 Sekunden mittels einer mit Stickstoff betriebenen Zweistoffdüse ein Gemisch aus 0,07 Gew.-% N-(2-Hydroxyethyl)-oxazolidinon, 0,07 Gew.-% 1,3-Propandiol, 1,50 Gew.-% Aluminiumhydroxid/Milchsäureamid-Komplex aus Beispiel 2 (ca. 25gew.-%ige wässrige Lösung), 0,30 Gew.-% Propylenglykol, 1,00 Gew.-% Isopropanol und 1,00 Gew.-% Wasser, jeweils bezogen auf das Grundpolymer, aufgesprüht und noch 5 Minuten nachgerührt (60 U/min). Anschließend wurde unter Rühren der Reaktormantel mittels Heizflüssigkeit aufgeheizt. Die Heizung wurde so nachgeführt, dass das Produkt möglichst schnell die Zieltemperatur von 180°C erreichte und dann dort stabil und unter Rühren getempert wurde. Der Reaktor wurde hierbei mit Stickstoff überlagert. Regelmäßig wurden dann zu den in der Tabelle angegebenen Zeiten (nach Beginn des Aufheizens) Proben entnommen und die Eigenschaften bestimmt. Die Ergebnisse sind in der Tabelle 1 zusammengefasst.

Beispiel 6

Es wurde verfahren wie unter Beispiel 5. Das im Schaufeltrockner vorgelegte Grundpolymer wurde vor dem Aufsprühen der Mischung auf 50°C aufgewärmt. Die Ergebnisse sind in der Tabelle 1 zusammengefasst.

Beispiel 7

Es wurde verfahren wie unter Beispiel 5. Das im Schaufeltrockner vorgelegte Grundpolymer wurde vor dem Aufsprühen der Mischung auf 50°C aufgewärmt. Statt des Aluminiumhydroxid/Milchsäureamid-Komplexes aus Beispiel 2 wurde der Aluminiumhydroxid/Milchsäureamid-Komplex aus Beispiel 3 (ca. 25gew.-%ige wässrige Lösung) eingesetzt. Die Ergebnisse sind in der Tabelle 1 zusammengefasst.

Beispiel 8

Es wurde verfahren wie unter Beispiel 5. Das im Schaufeltrockner vorgelegte Grundpolymer wurde vor dem Aufsprühen der Mischung auf 50°C aufgewärmt. Statt des Aluminiumhydroxid/Milchsäureamid-Komplexes aus Beispiel 2 wurde Aluminiumtrilaktat aus Beispiel 4 (ca. 25gew.-%ige wässrige Lösung) eingesetzt. Die Ergebnisse sind in der

Tabelle 1 zusammengefasst.

Tab. 1: Oberflächennachvernetzung

| Bsp. | Komplex | Temperatur [°C] | Zeit [min] | CRC 1g/gl | AUL0.7psi [g/g] | GBP [darcies] | FSR [g/gs] |
|---|---|---|---|---|---|---|---|
| 5 | Bsp. 2 | 144 | 20 | 37,1 | 7,4 | 0,2 | 0,45 |
| | | 179 | 40 | 36,9 | 8,0 | 0,4 | 0,43 |
| | | 188 | 60 | 36,5 | 21,2 | 9,4 | 0,28 |
| | | 178 | 80 | 30,4 | 22,0 | 29 | 0,23 |
| | | 180 | 100 | 28,0 | 20,3 | 39 | 0,23 |
| | | 183 | 120 | 28,2 | 20,4 | 40 | 0,17 |
| 6 | Bsp. 2 | 153 | 20 | 37,7 | 7,3 | 0,2 | 0,43 |
| | | 181 | 40 | 37,6 | 9,0 | 0,7 | 0,39 |
| | | 185 | 60 | 32,0 | 22,2 | 12 | 0,27 |
| | | 183 | 80 | 30,4 | 20,3 | 26 | 0,32 |
| | | 185 | 100 | 29,9 | 19,6 | 30 | 0,26 |
| | | 186 | 120 | 28,6 | 19,6 | 31 | 0,18 |
| 7 | Bsp. 3 | 158 | 20 | 38,9 | 7,1 | 0,1 | 0,30 |
| | | 185 | 40 | 38,5 | 9,0 | 0,2 | 0,32 |
| | | 187 | 60 | 31,5 | 23,5 | 6,3 | 0,28 |
| | | 188 | 80 | 30,3 | 21,5 | 10 | 0,24 |
| | | 185 | 100 | 29,0 | 20,5 | 12 | 0,21 |
| | | 186 | 120 | 28,9 | 20,5 | 16 | 0,16 |
| 8*) | Bsp. 4 | 158 | 20 | 37,9 | 7,2 | 0,0 | 0,44 |
| | | 185 | 40 | 34,6 | 11,5 | 0,2 | 0,32 |
| | | 188 | 60 | 32,1 | 23,3 | 2,3 | 0,31 |
| | | 187 | 80 | 28,6 | 22,9 | 7,8 | 0,22 |
| | | 188 | 100 | 28,0 | 21,5 | 11 | 0,26 |
| | | 182 | 120 | 24,2 | 20,4 | 15 | 0,19 |
| *) Vergleichsbeispiel | | | | | | | |

Der Ergebnisse zeigen, dass bei Verwendung der erfindungsgemäßen Komplexe bei vergleichbarer Zentrifugenretentionskapazität (CRC) eine höhere Gelbettpermeabilität (GBP) erreicht wird.

## Patentansprüche

1. Verfahren zur Herstellung wasserabsorbierender Polymerpartikel durch Polymerisation einer Monomerlösung oder-suspension, enthaltend

   i) mindestens ein ethylenisch ungesättigtes, säuregruppentragendes Monomer, das zumindest teilweise neutralisiert sein kann,
   ii) mindestens einen Vernetzer,
   iii) optional ein oder mehrere mit den unter i) genannten Monomeren copolymerisierbare ethylenisch ungesättigte Monomere und
   iv) optional ein oder mehrere wasserlösliche Polymere,
   wobei das erhaltene Polymergel getrocknet, gemahlen, klassiert, mit
   v) mindestens einem Oberflächennachvernetzer

   beschichtet und thermisch oberflächennachvernetzt wird, **dadurch gekennzeichnet, dass** die wasserabsorbierenden Polymerpartikel vor, während oder nach der thermischen Oberflächennachvernetzung mit mindestens einem Komplex, bestehend aus einem mehrwertigen Metallsalz der allgemeinen Formel (I)

$$M^n(X)_a(OH)_b \quad (I),$$

und einem 2-Hydroxycarbonsäureamid, beschichtet werden, worin

M mindestens ein mehrwertiges Metallkation,
X mindestens ein Säureanion,
a eine Zahl von 0 bis n/m, wobei m die Anzahl der negativen Ladungen des Säureanions und n die Anzahl der positiven Ladungen des mehrwertigen Metallkations ist, und
b eine Zahl 0 bis n

bedeuten.

2. Verfahren gemäß Anspruch 1, **dadurch gekennzeichnet, dass** das mehrwertige Metallkation ausgewählt wird aus der Gruppe $Al^{3+}$, $Ti^{4+}$ und $Zr^{4+}$.

3. Verfahren gemäß Anspruch 1 oder 2, **dadurch gekennzeichnet, dass** das Säureanion ausgewählt wird aus der Gruppe Acetat, Propionat, Glykolat, Laktat, Methansulfonat und Sulfat.

4. Verfahren gemäß einem der Ansprüche 1 bis 3, **dadurch gekennzeichnet, dass** das 2-Hydroxycarbonsäureamid ausgewählt wird aus der Gruppe Glykolsäureamid und Milchsäureamid.

5. Verfahren gemäß einem der Ansprüche 1 bis 4, **dadurch gekennzeichnet, dass** der Komplex von 0,5 bis 1,5 mol des 2-Hydroxycarbonsäureamids, bezogen auf das mehrwertige Metallkation, enthält.

6. Verfahren gemäß einem der Ansprüche 1 bis 5, **dadurch gekennzeichnet, dass** von 0,1 bis 1 Gew.-% des Komplexes, bezogen auf die wasserabsorbierenden Polymerpartikel, eingesetzt werden.

7. Verfahren gemäß einem der Ansprüche 1 bis 6, **dadurch gekennzeichnet, dass** der Komplex als wässrige Lösung eingesetzt wird.

8. Wasserabsorbierende Polymerpartikel, erhältlich nach einem Verfahren gemäß einem der Ansprüche 1 bis 7.

9. Wasserabsorbierende Polymerpartikel, enthaltend

a) mindestens ein polymerisiertes ethylenisch ungesättigtes, säuregruppentragendes Monomer, das zumindest teilweise neutralisiert sein kann,
b) mindestens einen polymerisierten Vernetzer,
c) optional ein oder mehrere mit den unter a) genannten Monomeren copolymerisierte ethylenisch ungesättigte Monomere,
d) optional ein oder mehrere wasserlösliche Polymere und
e) mindestens einen umgesetzten Oberflächennachvernetzer,

wobei die wasserabsorbierenden Polymerpartikel mit mindestens einem Komplex, bestehend aus einem mehrwertigen Metallsalz der allgemeinen Formel (I)

$$M^n(X)_a(OH)_b \quad (I),$$

und einem 2-Hydroxycarbonsäureamid, beschichtet sind, worin

M mindestens ein mehrwertiges Metallkation,
X mindestens ein Säureanion,
a eine Zahl von 0 bis n/m, wobei m die Anzahl der negativen Ladungen des Säureanions und n die Anzahl der positiven Ladungen des mehrwertigen Metallkations ist, und
b eine Zahl 0 bis n

bedeuten.

10. Polymerpartikel gemäß Anspruch 9, wobei das mehrwertige Metallkation ausgewählt ist aus der Gruppe $Al^{3+}$, $Ti^{4+}$

und $Zr^{4+}$.

**11.** Polymerpartikel gemäß Anspruch 9 oder 10, wobei das Säureanion ausgewählt ist aus der Gruppe Acetat, Propionat, Glykolat, Laktat, Methansulfonat und Sulfat.

**12.** Polymerpartikel gemäß einem der Ansprüche 9 bis 11, wobei das 2-Hydroxycarbonsäureamid ausgewählt ist aus der Gruppe Glykolsäureamid und Milchsäureamid.

**13.** Polymerpartikel gemäß einem der Ansprüche 10 bis 12, wobei der Komplex von 0,5 bis 1,5 mol des 2-Hydroxycarbonsäureamids, bezogen auf das mehrwertige Metallkation, enthält.

**14.** Polymerpartikel gemäß einem der Ansprüche 10 bis 13, wobei die wasserabsorbierenden Polymerpartikel von 0,1 bis 1 Gew.-% des Komplexes enthalten.

**15.** Hygieneartikel, enthaltend wasserabsorbierende Polymerpartikel gemäß einem der Ansprüche 10 bis 14.

**Claims**

**1.** A process for producing water-absorbing polymer particles by polymerizing a monomer solution or suspension comprising

i) at least one ethylenically unsaturated monomer which bears acid groups and may be at least partly neutralized,
ii) at least one crosslinker,
iii)optionally one or more ethylenically unsaturated monomers copolymerizable with the monomers mentioned under i) and
iv) optionally one or more water-soluble polymers, and drying, grinding and classifying the resulting polymer gel, coating it with
v) at least one surface postcrosslinker

and thermally surface postcrosslinking it, wherein the water-absorbing polymer particles are coated before, during or after the thermal surface postcrosslinking with at least one complex consisting of a polyvalent metal salt of the general formula (I)

$$M^n(X)_a(OH)_b \qquad (I)$$

and a 2-hydroxycarboxamide, in which

M is at least one polyvalent metal cation,
X is at least one acid anion,
a is a number from 0 to n/m, where m is the number of negative charges of the acid anion and n is the number of positive charges of the polyvalent metal cation, and
b is a number from 0 to n.

**2.** The process according to claim 1, wherein the polyvalent metal cation is selected from the group of $Al^{3+}$, $Ti^{4+}$ and $Zr^{4+}$.

**3.** The process according to claim 1 or 2, wherein the acid anion is selected from the group of acetate, propionate, glycolate, lactate, methanesulfonate and sulfate.

**4.** The process according to any of claims 1 to 3, wherein the 2-hydroxycarboxamide is selected from the group of glycolamide and lactamide.

**5.** The process according to any of claims 1 to 4, wherein the complex comprises from 0.5 to 1.5 mol of the 2-hydroxycarboxamide, based on the polyvalent metal cation.

**6.** The process according to any of claims 1 to 5, wherein from 0.1 to 1% by weight of the complex is used, based on the water-absorbing polymer particles.

7. The process according to any of claims 1 to 6, wherein the complex is used in the form of an aqueous solution.

8. Water-absorbing polymer particles obtainable by a process according to any of claims 1 to 7.

9. Water-absorbing polymer particles comprising

a) at least one polymerized ethylenically unsaturated monomer which bears acid groups and may be at least partly neutralized,
b) at least one polymerized crosslinker,
c) optionally one or more ethylenically unsaturated monomers copolymerized with the monomers mentioned under a),
d) optionally one or more water-soluble polymers and
e) at least one reacted surface postcrosslinker,

said water-absorbing polymer particles having been coated with at least one complex consisting of a polyvalent metal salt of the general formula (I)

$$M^n(X)_a(OH)_b \qquad (I)$$

and a 2-hydroxycarboxamide, in which

M is at least one polyvalent metal cation,
X is at least one acid anion,
a is a number from 0 to n/m, where m is the number of negative charges of the acid anion and n is the number of positive charges of the polyvalent metal cation, and
b is a number from 0 to n.

10. Polymer particles according to claim 9, wherein the polyvalent metal cation is selected from the group of $Al^{3+}$, $Ti^{4+}$ and $Zr^{4+}$.

11. Polymer particles according to claim 9 or 10,
wherein the acid anion is selected from the group of acetate, propionate, glycolate, lactate, methanesulfonate and sulfate.

12. Polymer particles according to any of claims 9 to 11, wherein the 2-hydroxycarboxamide is selected from the group of glycolamide and lactamide.

13. Polymer particles according to any of claims 10 to 12, wherein the complex comprises from 0.5 to 1.5 mol of the 2-hydroxycarboxamide, based on the polyvalent metal cation.

14. Polymer particles according to any of claims 10 to 13, wherein the water-absorbing polymer particles comprise from 0.1 to 1% by weight of the complex.

15. A hygiene article comprising water-absorbing polymer particles according to any of claims 10 to 14.

**Revendications**

1. Procédé de fabrication de particules polymères absorbant l'eau par polymérisation d'une solution ou suspension de monomères, contenant :

i) au moins un monomère éthyléniquement insaturé, portant des groupes acides, qui peut être neutralisé au moins en partie,
ii) au moins un agent de réticulation,
iii) éventuellement un ou plusieurs monomères éthyléniquement insaturés copolymérisables avec les monomères indiqués en i), et
iv) éventuellement un ou plusieurs polymères solubles dans l'eau,
le gel polymère obtenu étant séché, broyé, classifié, revêtu avec

v) au moins un agent de post-réticulation de surface, et post-réticulé thermiquement en surface, **caractérisé en ce que** les particules polymères absorbant l'eau sont revêtues avant, pendant ou après la post-réticulation de surface thermique avec au moins un complexe, constitué par un sel métallique polyvalent de formule générale (I)

$$M^n(X)_a(OH)_b \qquad (I)$$

et un amide d'acide 2-hydroxycarboxylique,

M signifiant au moins un cation métallique polyvalent,
X signifiant au moins un anion acide,
a signifiant un nombre de 0 à n/m, m étant le nombre de charges négatives de l'anion acide et n étant le nombre de charges positives du cation métallique polyvalent, et
b signifiant un nombre de 0 à n.

**2.** Procédé selon la revendication 1, **caractérisé en ce que** le cation métallique polyvalent est choisi dans le groupe constitué par $Al^{3+}$, $Ti^{4+}$ et $Zr^{4+}$.

**3.** Procédé selon la revendication 1 ou 2, **caractérisé en ce que** l'anion acide est choisi dans le groupe constitué par acétate, propionate, glycolate, lactate, méthane-sulfonate et sulfate.

**4.** Procédé selon l'une quelconque des revendications 1 à 3, **caractérisé en ce que** l'amide de l'acide 2-hydroxycarboxylique est choisi dans le groupe constitué par un amide de l'acide glycolique et un amide de l'acide lactique.

**5.** Procédé selon l'une quelconque des revendications 1 à 4, **caractérisé en ce que** le complexe contient de 0,5 à 1,5 mole de l'amide de l'acide 2-hydroxycarboxylique, par rapport au cation métallique polyvalent.

**6.** Procédé selon l'une quelconque des revendications 1 à 5, **caractérisé en ce que** de 0,1 à 1 % en poids du complexe, par rapport aux particules polymères absorbant l'eau, est utilisé.

**7.** Procédé selon l'une quelconque des revendications 1 à 6, **caractérisé en ce que** le complexe est utilisé sous la forme d'une solution aqueuse.

**8.** Particules polymères absorbant l'eau, pouvant être obtenues par un procédé selon l'une quelconque des revendications 1 à 7.

**9.** Particules polymères absorbant l'eau, contenant :

a) au moins un monomère éthyléniquement insaturé, portant des groupes acides, polymérisé, qui peut être neutralisé au moins en partie,
b) au moins un agent de réticulation polymérisé,
c) éventuellement un ou plusieurs monomères éthyléniquement insaturés copolymérisés avec les monomères indiqués en a),
d) éventuellement un ou plusieurs polymères solubles dans l'eau, et
e) au moins un agent de post-réticulation de surface transformé,

les particules polymères absorbant l'eau étant revêtues avec au moins un complexe, constitué par un sel métallique polyvalent de formule générale (I)

$$M^n(X)_a(OH)_b \qquad (I)$$

et un amide d'acide 2-hydroxycarboxylique,

M signifiant au moins un cation métallique polyvalent,
X signifiant au moins un anion acide,
a signifiant un nombre de 0 à n/m, m étant le nombre de charges négatives de l'anion acide et n étant le nombre de charges positives du cation métallique polyvalent, et
b signifiant un nombre de 0 à n.

10. Particules polymères selon la revendication 9, dans lesquelles le cation métallique polyvalent est choisi dans le groupe constitué par $Al^{3+}$, $Ti^{4+}$ et $Zr^{4+}$.

11. Particules polymères selon la revendication 9 ou 10, dans lesquelles l'anion acide est choisi dans le groupe constitué par acétate, propionate, glycolate, lactate, méthane-sulfonate et sulfate.

12. Particules polymères selon l'une quelconque des revendications 9 à 11, dans lesquelles l'amide de l'acide 2-hydroxycarboxylique est choisi dans le groupe constitué par un amide de l'acide glycolique et un amide de l'acide lactique.

13. Particules polymères selon l'une quelconque des revendications 10 à 12, dans lesquelles le complexe contient 0,5 à 1,5 mole de l'amide de l'acide 2-hydroxycarboxylique, par rapport au cation métallique polyvalent.

14. Particules polymères selon l'une quelconque des revendications 10 à 13, dans lesquelles les particules polymères absorbant l'eau contiennent de 0,1 à 1 % en poids du complexe.

15. Article d'hygiène, contenant des particules polymères selon l'une quelconque des revendications 10 à 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20050256757 A
- WO 2004069915 A2
- WO 2009041731 A1
- US 20100247916 A
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 0343427 A1
- DE 10319462 A1
- DE 19941423 A1
- EP 0686650 A1
- WO 200145758 A1
- WO 2001038402 A1
- EP 0955086 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- EP 1199327 A1
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- EP 0534228 A1
- EP 1191051 A1
- WO 2002060983 A1
- US 20030181115 A
- US 20040019342 A
- EP 0377199 A1
- EP 0445641 A1
- US 5026806 A
- EP 0655465 A1
- EP 0377191 A1
- US 20040167486 A
- US 20040071363 A
- US 20050097025 A
- US 20070156108 A
- US 20080125735 A
- EP 1917940 A2
- EP 1913912 A1
- EP 1913913 A2
- EP 1913914 A1
- EP 1911425 A2
- EP 1911426 A2
- EP 1447067 A1
- EP 1813237 A2
- EP 1813236 A2
- EP 1808152 A2
- EP 1447066 A1
- WO 2008155722 A2
- WO 2008155702 A1
- WO 2008155711 A1
- WO 2008155710 A1
- WO 2008155701 A2
- WO 2008155699 A1
- US 20060004336 A
- US 20070135785 A
- US 20050137085 A


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 71-103